# EUROPÄISCHE PATENTSCHRIFT

(11) **EP 2 575 697 B1**
(45) Veröffentlichungstag und Bekanntmachung des Hinweises auf die Patenterteilung: **11.06.2014**
(21) Anmeldenummer: 11726699.9
(22) Anmeldetag: 25.05.2011
(51) Int. Cl.: A61F 2/95, A61B 17/12

(54) **VORRICHTUNG ZUM EINBRINGEN EINES IMPLANTATS**
DEVICE FOR INSERTING AN IMPLANT
DISPOSITIF D'INTRODUCTION D'IMPLANT

(30) Priorität: 28.05.2010 DE 102010021947
(43) Veröffentlichungstag der Anmeldung: 10.04.2013
(73) Patentinhaber: Phenox GmbH, 44801 Bochum (DE)
(72) Erfinder: APORTA, Carsten, 44805 Bochum (DE); ASCHERFELD, Jörg, 45529 Hattingen (DE); HANNES, Ralf, 44137 Dortmund (DE); MONSTADT, Hermann, 44797 Bochum (DE)
(74) Vertreter: Schöneborn, Holger
(86) Internationale Anmeldenummer: PCT/EP2011/002592
(87) Internationale Veröffentlichungsnummer: WO 2011/147567

(56) Entgegenhaltungen:
- US-A1- 2006 282 112
- BRUCE G. POUND: "Electrochemical behavior of cobalt-chromium alloys in a simulated physiological solution", JOURNAL OF BIOMEDICAL MATERIALS RESEARCH PART A, Bd. 94A, Nr. 1, 2. Februar 2010 (2010-02-02), Seiten 93-102, XP55005165, ISSN: 1549-3296, DOI: 10.1002/jbm.a.32684

## Beschreibung

Die Erfindung betrifft eine Vorrichtung, umfassend ein endovaskuläres Implantat, das zur Einbringung in Blutgefäße oder Körperhohlräume des menschlichen oder tierischen Körpers vorgesehen ist, und eine Einführhilfe, wobei das Implantat und die Einführhilfe über ein Ablöseelement miteinander verbunden sind, welches elektrolytisch korrodierbar ausgebildet ist, so dass nach Einbringen des Implantats in den Körper durch Anlegen einer Spannung eine zumindest teilweise Auflösung des Ablöseelements und eine Ablösung des Implantats von der Einführhilfe erfolgt.

Der Einsatz endovaskulärer Techniken zur Okklusion von Körperhohlräumen oder Gefäßen wie Arterien, Venen, Eileitern oder vaskulären Fehlbildungen (z. B. vaskuläre Aneurysmen) ist bekannter Stand der Technik. Hierbei werden z. B. sogenannte Okklusionswendeln mit Hilfe eines als Einführhilfe dienenden endovaskulären Führungsdrahtes durch einen Katheter in den zu okkludierenden Hohlraum eingeführt und dort deponiert. Ebenfalls bekannt ist die Einbringung anderer Implantate, beispielsweise Stents.

Zur Abtrennung des Implantats von der Einführhilfe sind aus dem Stand der Technik verschiedene Verfahren bekannt. Neben mechanischen Verfahren hat sich besonders die elektrolytische Ablösung von Edelstahldrahtspitzen bewährt, wie sie erstmalig bei der Elektrokoagulation durch Thompson et al. sowie McAlister et al. im Jahre 1979 beschrieben wurde (Radiology 133:335-340, Nov. 1979; AJR 132:998-1000, Juni 1979). Hierauf aufbauend beschreibt auch die EP 0 484 468 B1 eine Vorrichtung zur Implantation von Okklusionswendeln basierend auf einer elektrolytischen Ablösung.

Ähnliche Vorrichtungen offenbart auch die US 2006/ 0 282 112 A1. Darüber hinaus sind diesem Dokument verschiedene Materialien für den katheter zu entnehmen, darunter auch MP35-M, einer Legierung, die u.a. 35% N:, 35% Co, 20% Cr und 9,75% Mo enthälten.

Unabhängig von der Art des konkret einzubringenden Implantates ist es für den behandelnden Arzt stets von Bedeutung, die Ablösezeiten möglichst kurz zu halten, damit beispielsweise während der Ablösung keine Verlagerung des Implantats oder andere unvorhersehbare Dinge erfolgen. Aus diesem Grund wurden bereits in der Vergangenheit für das zwischen Implantat und Einführhilfe angeordnete Ablöseelement unterschiedliche Materialien verwendet, die bei Anlegen einer Spannung eine rasche Auflösung ermöglichen. In der WO 03/017852 A1 beispielsweise wird ein Edelstahl verwendet, der einem Vorkorrosionsprozess über eine Wärmebehandlung unterzogen wurde.

Die US 2006/082 112 A1 bildet dem Oberbegriff des Anspruchs 1.

Es stellt sich somit die Aufgabe, ausgehend von einer Vorrichtung der eingangs beschriebenen Art Mittel zur Verfügung zu stellen, die die Ablösezeiten des Implantates von der Einführhilfe weiter verringern.

Diese Aufgabe wird erfindungsgemäß gelöst durch eine Vorrichtung, umfassend ein endovaskuläres Implantat, das zur Einbringung in Blutgefäße oder Körperhohlräume des menschlichen oder tierischen Körpers vorgesehen ist, und eine Einführhilfe, wobei das Implantat und die Einführhilfe über ein Ablöseelement miteinander verbunden sind, welches elektrolytisch korrodierbar ausgebildet ist, so dass nach Einbringen des Implantats in den Körper durch Anlegen einer Spannung eine zumindest teilweise Auflösung des Ablöseelementes und eine Ablösung des Implantats von der Einführhilfe erfolgt, wobei das Ablöseelement aus einer Cobalt-Chrom-Legierung, enthaltend mindestens 20 Gew.-% Cobalt und 10 bis 40 Gew.-% Chrom, gefertigt ist.

Überraschend hat sich herausgestellt, dass Ablöseelemente, die auf Cobalt-Chrom-Legierungen basieren, außerordentlich kurze Ablösezeiten bei Anlegen einer Spannung ermöglichen. Diese liegen in der Regel unter 30 s (2 V, 2 mA). Auch Ablösezeiten von lediglich 5 s sind möglich, wobei die Ablösezeiten selbstverständlich auch von der Dicke des Ablöseelements abhängen. Gegenüber dem Stand der Technik, der z. B. in der WO 2005/070308 A2 von 20 bis 40 s bei ansonsten gleichen Bedingungen spricht, stellt dies eine deutliche Verbesserung dar.

Als Cobalt-Chrom-Legierungen können solche eingesetzt werden, wie sie als Stellite^{®} von der Fa. Deloro vertrieben werden. Es handelt sich hierbei um Cobalt-Chrom-Legierungen, die Anteile von Wolfram, Nickel, Molybdän, Eisen und anderen Elementen haben können. Stellite^{®} sind sehr beständig gegen Verschleiß, weshalb sie insbesondere für Bauteile eingesetzt werden, die hohen Belastungen ausgesetzt sind. Um so überraschender war die Erkenntnis, dass sich aus Stelliten^{®} gefertigte Ablöseelemente elektrolytisch besonders rasch auflösen lassen.

Der Gehalt Cobalt liegt vorzugsweise bei max. 70 Gew.-%, insbesondere ist ein Gehalt zwischen 30 und 60 Gew.-% vorteilhaft. Der Chromgehalt kann insbesondere 15 bis 30 Gew.-% betragen.

Weitere Elemente können die Eigenschaften des Ablöseelements verbessern. So ist eine Legierung mit 4 bis 12 Gew.-% Molybdän und/oder 5 bis 20 Gew.% Wolfram von Vorteil.

Der Gehalt von Nickel, Mangan oder Eisen kann bei Bedarf deutlich höher liegen und im Falle von Mangan und Eisen bis zu 30 Gew.-% betragen. Der Nickelgehalt kann zwischen 8 und 40 Gew.-% liegen.

Beispiele für verwendbare Cobalt-Chrom-Legierungen sind solche, wie sie unter den Namen Elgiloy^{®} oder Phynox^{®} vertrieben werden. Die deutsche Werkstoffnummer lautet 2.4711. Die Legierung enthält 40 Gew.-% Cobalt, 20 Gew.-% Chrom, 16 Gew.-% Eisen, 15 Gew.-% Nickel und 7 Gew.-% Molybdän sowie ggf. in geringen Mengen Mangan, Kohlenstoff, Silicium, Phosphor, Schwefel und Beryllium.

Eine andere verwendbare Legierung ist Stellite^{®}25. Diese Legierung enthält u. a. ca. 50 Gew.-% Cobalt, 20 Gew.-% Chrom, 10 Gew.-% Nickel und 15 Gew.-% Wolfram. Ein weiteres Beispiel stellt Stellite^{®}21 dar mit ca. 63 Gew.-% Cobalt, 28 Gew.-% Chrom und 6 Gew.-% Molybdän.

Bei der Einführhilfe handelt es sich vorzugsweise um einen herkömmlichen Führungsdraht, wie er sich bewährt hat, um Okklusionswendeln oder Stents durch einen Katheter an ihren Bestimmungsort zu bringen. Die Einführhilfe schließt sich proximal an das Implantat an, d. h. in der Richtung, von der aus der Arzt das Implantat vorschiebt.

Das Implantat kann eine Okklusionswendel oder ein Stent sein. Darüber hinaus kann das Implantat auch ein Stent-ähnliches System zur Beeinflussung des Blutstroms sein, das entweder geflochten, per Laserschneiden aus einer Metall- oder Kunststofffolie oder durch geeignete Kunststoffmembranen gestaltet ist. Grundsätzlich ist die Erfindung jedoch für sämtliche Arten von endovaskulären Implantaten einsetzbar, die mit Hilfe einer Einführhilfe an ihren Bestimmungsort gebracht und dort von der Einführhilfe abgelöst werden sollen. Unter Implantaten werden auch solche Objekte verstanden, die nur wahlweise ablösbar ausgebildet sind und je nach Verlauf der Behandlung im Körper verbleiben oder wieder entfernt werden. Ein Beispiel hierfür sind ablösbare Stent-Retriever-Systeme zum Entfernen oder Verdrängen von Thromben.

Vorteilhafterweise besteht das Ablöseelement aus einem oder mehreren Drähten. Über die Drähte können Schub- oder Zugkräfte gut weitergegeben werden, so dass dem behandelnden Arzt sowohl ein einfacher Vorschub, aber auch das Zurückziehen des Implantats möglich ist. Eine einfache Positionierbarkeit ist wichtig, damit der Arzt das Implantat exakt an den gewünschten Bestimmungsort bringen kann.

Der oder die Drähte können über einen runden, eckigen, ovalen oder rohrförmigen Querschnitt verfügen. Dabei sind auch beliebige Kombinationen möglich, beispielsweise aus runden und eckigen Drähten. Die Herstellung der Drähte erfolgt in der Regel durch Herunterziehen des Rohmaterials auf den entsprechenden Endquerschnitt. Durch den Ziehprozess lassen sich der Gefügeaufbau und somit die Ablöseeigenschaften des Ablöseelements steuern. Durch Variation des Querschnitts und der Form der Drähte können diese an den Einsatzzweck optimal angepasst werden.

Um die Ablösung weiter zu vereinfachen, ist es möglich, an den Ablöseelementen eine Oberflächenbehandlung durchzuführen. Eine Aufrauung der Oberfläche beispielsweise bewirkt eine schnellere Auflösung, da die Oberfläche des Ablöseelementes vergrößert wird. Ebenfalls bekannt sind weitere Möglichkeiten, ein Ablöseelement durch eine Zusatzbehandlung zu einer schnelleren Auflösung zu bewegen, beispielsweise aus der bereits erwähnten WO 03/017852 A1, auf die insofern Bezug genommen wird. Beispielsweise kann das Ablöseelement einer Wärmebehandlung im Sinne eines Ausscheidungsprozesses unterzogen werden, durch den das Metall in seinem Gefüge so verändert wird, dass es sich bei Anlegen einer elektrischen Spannung in einem Elektrolyten besonders rasch zersetzt. Eine solche Wärmebehandlung ist mit Hilfe eines Lasers, in einem Ofen oder mittels einer Induktionsspule möglich. Die Abkühlung erfolgt zweckmäßigerweise relativ rasch in Form einer Abschreckung. Auf diese Weise werden Gefügezustände erzeugt, die die elektrolytische Auflösung fördern.

Cobalt zeigt beim Erwärmen und Abkühlen eine reversible allotrope Phasentransformation. Bei hoher Temperatur ist die kubisch-flächenzentrierte Phase (α-Cobalt) stabil, die beim Abkühlen bei ca. 420 °C in das hexagonale ε-Cobalt übergeht. Während ε-Cobalt sehr schlecht umformbar ist, ist die metastabile α-Phase duktiler. Die Umwandlungstemperatur zwischen den beiden Phasen lässt sich durch die Wahl unterschiedlicher Legierungszusätze variieren. Grundsätzlich stabilisieren Zusätze von Chrom, Molybdän oder Wolfram die hexagonale Phase und verringern die Stapelfehlenergie, während die Elemente Eisen, Nickel und Mangan die kubische Phase begünstigen und die Stapelfehlenergie erhöhen. Nach dem Lösungsglühen besteht die Legierung normalerweise in erster Linie aus α-Cobalt, anschließend wird durch Kaltumformung (z. B. Drahtziehen) und/oder Ausscheidungsprozesse der Gehalt an ε-Cobalt erhöht. Durch eine Ausscheidungshärtung können aus der Legierung intermetallische Phasen ausgeschieden werden, wodurch die Korrosionsanfälligkeit der Legierung erhöht wird. Letztlich sollte die Legierung vorwiegend metastabil als α-Cobalt vorliegen, jedoch auch einen bestimmten Gehalt an ε-Cobalt aufweisen.

Ebenfalls möglich sind andere Arten der Zusatzbehandlung wie eine Vorkorrosion, beispielsweise über eine Anätzung. Des Weiteren ist es möglich, die Ablösung durch konstruktionsbedingte Bildung lokaler Korrosionselemente passiv zu beschleunigen. Derartige Korrosionselemente bilden sich bei benachbarter Anordnung unterschiedlich edler Metalle, d. h. das Ablöseelement ist aus einem unedleren Metall gefertigt als das Implantat und/oder die Einführhilfe.

Die Auflösung des Ablöseelements erfolgt durch Anlegen einer elektrischen Spannung. Dabei kann es sich sowohl um Wechselstrom als auch um Gleichstrom handeln, wobei eine geringe Stromstärke (< 3 mA) ausreicht. Das Ablöseelement stellt dabei die Anode dar, an der die Oxidation und Auflösung des Metalls stattfindet.

Zur Verbesserung der aktiven Beeinflussbarkeit der Auflösung kann es sinnvoll sein, das Ablöseelement konstruktiv so auszulegen, dass die oben beschriebenen lokalen Korrosionselemente vermieden werden. Dies kann z. B. durch Isolierung des Ablöseelements gegenüber angrenzenden Bereichen der Vorrichtung z. B. durch isolierende Klebeverbindungen zwischen Ablöseelement und Implantat erfolgen.

Die elektrolytische Ablösung erfolgt, indem mit Hilfe einer Spannungsquelle eine elektrische Spannung an das Ablöseelement angelegt wird. Das Ablöseelement dient dabei als Anode, während die Kathode auf der Körperoberfläche positioniert wird. Selbstverständlich muss das Ablöseelement entsprechend elektrisch leitend, insbesondere über die Einführhilfe, mit der Spannungsquelle verbunden sein. Die Einführhilfe muss in diesem Fall auch selbst elektrisch leitfähig ausgebildet sein. Da der sich einstellende Korrosionsstrom von der Fläche der Kathode gesteuert wird, sollte die Fläche der Kathode deutlich größer gewählt werden als die Fläche der Anode. In gewissem Maße lässt sich die Auflösungsgeschwindigkeit des Ablöseelementes durch Einstellung der Kathodenfläche im Verhältnis zur Anodenfläche steuern. Die Erfindung betrifft entsprechend auch eine Vorrichtung, die eine Spannungsquelle sowie ggf. eine auf der Körperoberfläche platzierbare Elektrode umfasst.

Alternativ oder zusätzlich können weitere Maßnahmen ergriffen werden, um die Auf- bzw. Ablösung des Ablöseelements zu unterstützen. In diesem Zusammenhang sei die Verwendung von Lichtwellen, Schall (Ultraschall) oder magnetischer Kräfte erwähnt.

Zusätzlich ist es zweckmäßig, wenn durch das Implantat ein Sicherungsmittel verläuft. Derartige Sicherungsmittel haben den Vorteil, dass im Falle einer fehlerhaften Positionierung des Implantates das notwendige Zurückziehen in den Katheter deutlich sicherer wird. Das Zurückziehen beispielsweise einer Okklusionswendel ohne Sicherungsmittel birgt die Gefahr, dass Teile der Wendel durch Zug- oder Torsionsbeanspruchung auseinandergezogen und so irreversibel plastisch deformiert werden. Im Extremfall kann die Wendel reißen oder brechen, was eine lebensgefährliche Embolie nach sich ziehen kann. Beschrieben wurden sowohl Sicherungsmittel aus flexiblen Polymerfäden als auch aus Materialien mit Formgedächtniseigenschaften.

Ebenfalls bereits beschrieben wurden Vorrichtungen, bei denen mehrere Ablöseelemente vorgesehen sind, so dass ggf. variabel dimensionierte Längen des Implantates am Bestimmungsort platziert werden können. Dies erlaubt es beispielsweise, Okklusionswendeln in genau der richtigen Länge im Aneurysma abzusetzen. In diesem Zusammenhang sei auf die WO 01/32085 A1 verwiesen.

Die Verwendung derartiger, an mehreren Stellen elektrolytisch korrodierbarer Implantate beruht auf der Erkenntnis, dass bei Anlegen eines Stroms an eine solche Vorrichtung sich spezifisch die dem distalen Ende des Katheters am nächsten liegende Ablösestelle durch Elektrolyse auflöst. Dies ist darauf zurückzuführen, dass einerseits die sich im Katheter befindenden Ablösestellen durch den Katheter vom ionischen Medium isoliert sind und deshalb keiner Elektrolyse unterliegen können und andererseits die Stromdichte aufgrund des nach distal zunehmenden Widerstands von proximal nach distal abnimmt. Die sich nach distal als erstes an das distale Katheterende anschließende elektrolytisch korrodierbar ausgebildete Stelle ist deshalb am stärksten elektrolytischen Prozessen unterworfen und löst sich bevorzugt auf.

Für die Ausbildung der Implantate hat sich insbesondere die Verwendung von Platin oder Platinlegierungen bewährt. Diese weisen unter anderem den Vorteil auf, dass sie röntgendicht sind und somit die Einbringung des Implantates einfach visualisiert werden kann. Die für das Ablöseelement verwendeten Cobalt-Chrom-Legierungen sind i. d. R. MR-kompatibel und ermöglichen daher ebenfalls eine Visualisierung.

Die erfindungsgemäße Vorrichtung kann auch direkt in Kombination mit einem Mikrokatheter vorliegen, durch den das Implantat mit Hilfe der Einführhilfe an seinen Bestimmungsort gebracht wird. Der verwendete Katheter und das Implantat sollten dabei hinsichtlich ihrer Dimensionen aufeinander abgestimmt sein. Ggf. kann der Katheter dabei auch einen Zwang auf das Implantat ausüben, der bewirkt, dass das Implantat erst nach Befreiung vom Zwang eine ihm zuvor aufgeprägte Sekundärstruktur einnimmt. Zusätzlich ist der Katheter darüber hinaus mit röntgendichten Markierungen versehen, die die Positionierung im Zielbereich mit Hilfe bekannter bildgebender Verfahren ermöglicht.

Typischerweise weist das Ablöseelement eine Länge von 0,05 bis 0,5 mm, insbesondere ca. 0,2 mm, und einen Durchmesser von 0,04 bis 0,5 mm, insbesondere ca. 0,1 mm, auf.

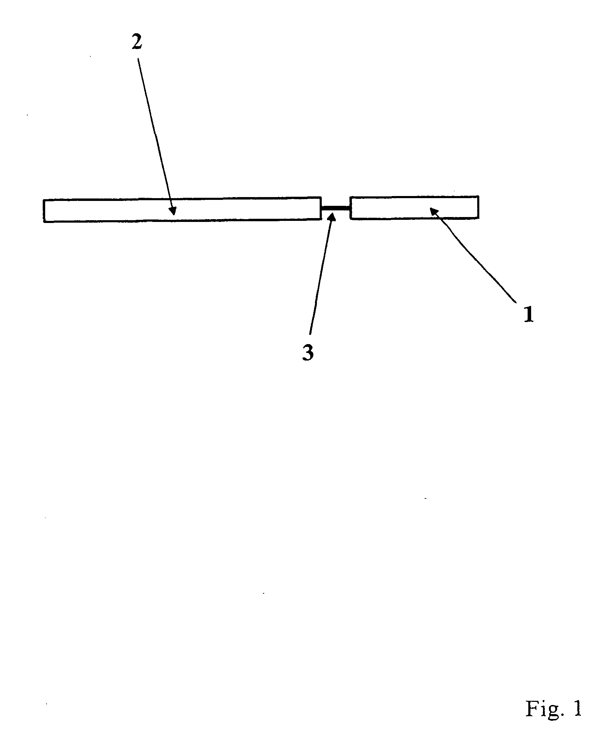

Die Erfindung wird anhand der beigefügten Figur 1 näher erläutert. Diese zeigt den schematischen Aufbau der erfindungsgemäßen Vorrichtung.

Die Vorrichtung setzt sich aus einem Implantat 1, einer Einführhilfe 2 und einem Ablöseelement 3 zusammen. Dabei befindet sich die Einführhilfe 2 am proximalen, das Implantat 1 am distalen Ende, d. h. in Vorschubrichtung der Vorrichtung. Das Implantat 1 wird durch Vorschieben der Einführhilfe 2 in einem hier nicht dargestellten Katheter an seinen Bestimmungsort gebracht.

## Patentansprüche

1. Vorrichtung umfassend ein endovaskuläres Implantat (1), das zur Einbringung in Blutgefäße oder Körperhohlräume des menschlichen oder tierischen Körpers vorgesehen ist, und eine Einführhilfe (2), wobei das Implantat (1) und die Einführhilfe (2) über ein Ablöseelement (3) miteinander verbunden sind, welches elektrolytisch korrodierbar ausgebildet ist, so dass nach Einbringen des Implantats (1) in den Körper durch Anlegen einer Spannung eine zumindest teilweise Auflösung des Ablöseelementes (3) und eine Ablösung des Implantats (1) von der Einführhilfe (2) erfolgt, **dadurch gekennzeichnet, dass** das Ablöseelement (3) aus einer Cobalt-Chrom-Legierung, enthaltend mindestens 20 Gew.-% Cobalt und 10 bis 40 Gew.-% Chrom, gefertigt ist.

2. Vorrichtung nach Anspruch 1, **dadurch gekennzeichnet, dass** die Cobalt-Chrom-Legierung bis zu 70 Gew.-% Cobalt enthält.

3. Vorrichtung nach Anspruch 2, **dadurch gekennzeichnet, dass** die Cobalt-Chrom-Legierung 30 bis 60 Gew.-% Cobalt enthält.

4. Vorrichtung nach einem der Ansprüche 1 bis 3, **dadurch gekennzeichnet, dass** die Cobalt-Chrom-Legierung 15 bis 30 Gew.-% Chrom enthält.

5. Vorrichtung nach einem der Ansprüche 1 bis 4, **dadurch gekennzeichnet, dass** die Cobalt-Chrom-Legierung 4 bis 12 Gew.-% Molybdän enthält.

6. Vorrichtung nach einem der Ansprüche 1 bis 5, **dadurch gekennzeichnet, dass** die Cobalt-Chrom-Legierung 8 bis 40 Gew.-% Nickel enthält.

7. Vorrichtung nach einem der Ansprüche 1 bis 6, **dadurch gekennzeichnet, dass** die Cobalt-Chrom-Legierung 5 bis 20 Gew.-% Wolfram enthält.

8. Vorrichtung nach einem der Ansprüche 1 bis 7, **dadurch gekennzeichnet, dass** die Cobalt-Chrom-Legierung bis zu 30 Gew.-% Mangan enthält.

9. Vorrichtung nach einem der Ansprüche 1 bis 8, **dadurch gekennzeichnet, dass** die Cobalt-Chrom-Legierung bis zu 30 Gew.-% Eisen enthält.

10. Vorrichtung nach einem der Ansprüche 1 bis 9, **dadurch gekennzeichnet, dass** das Implantat (1) eine Okklusionswendel, ein Stent, ein ablösbares Stent-Retriever-System oder ein Stent-ähnliches System zur Beeinflussung des Blutstroms ist.

11. Vorrichtung nach einem der Ansprüche 1 bis 10, **dadurch gekennzeichnet, dass** das Ablöseelement (3) aus einem oder mehreren Drähten besteht.

12. Vorrichtung nach einem der Ansprüche 1 bis 11, **dadurch gekennzeichnet, dass** das Ablöseelement (3) eine raue Oberfläche aufweist.

13. Vorrichtung nach einem der Ansprüche 1 bis 12, **dadurch gekennzeichnet, dass** das Ablöseelement (3) vorkorrodiert ist.

14. Vorrichtung nach einem der Ansprüche 1 bis 13, **dadurch gekennzeichnet, dass** das Ablöseelement (3) oder die zur Herstellung des Ablöseelementes (3) dienende Legierung einer Wärmebehandlung unterzogen ist.

15. Vorrichtung nach einem der Ansprüche 1 bis 14, **dadurch gekennzeichnet, dass** die Vorrichtung in Kombination mit einem Mikrokatheter vorliegt.

## Claims

1. Device comprising an endovascular implant (1) for the introduction into blood vessels or body cavities of the human or animal body and an insertion aid (2), wherein the implant (1) and the insertion aid (2) being connected to each other via a detachment element (3) designed to be electrolytically corrodible so that after the implant (1) has been inserted into the body and a voltage has been applied an at least partial dissolution of the detachment element (3) takes place causing the implant (1) to be separated from the insertion aid (2), **characterized in that** the detachment element (3) is made of a cobalt-chrome alloy containing at least 20 % w/w cobalt and 10 to 40 % w/w chrome.

2. Device according to claim 1, **characterized in that** the cobalt-chrome alloy contains up to 70 % w/w of cobalt.

3. Device according to claim 2, **characterized in that** the cobalt-chrome alloy contains between 30 and 60 % w/w of cobalt.

4. Device according to any one of claims 1 to 3, **characterized in that** the cobalt-chrome alloy contains between 15 and 30 % w/w of chrome.

5. Device according to any one of claims 1 to 4, **characterized in that** the cobalt-chrome alloy contains between 4 and 12 % w/w of molybdenum.

6. Device according to any one of claims 1 to 5, **characterized in that** the cobalt-chrome alloy contains between 8 and 40 % w/w of nickel.

7. Device according to any one of claims 1 to 6, **characterized in that** the cobalt-chrome alloy contains between 5 and 20 % w/w of tungsten.

8. Device according to any one of claims 1 to 7, **characterized in that** the cobalt-chrome alloy contains up to 30 % w/w of manganese.

9. Device according to any one of claims 1 to 8, **characterized in that** the cobalt-chrome alloy contains up to 30 % w/w of iron.

10. Device according to any one of claims 1 to 9, **characterized in that** the implant (1) is an occlusion helix, a stent, a detachable stent retrieval system or a stent-like system devised so as to influence the flow of blood.

11. Device according to any one of claims 1 to 10, **characterized in that** the detachment element (3) consists of one or several wires.

12. Device according to any one of claims 1 to 11, **characterized in that** the detachment element (3) has a rough surface.

13. Device according to any one of claims 1 to 12, **characterized in that** the detachment element (3) is pre-corroded.

14. Device according to any one of claims 1 to 13, **characterized in that** the detachment element (3) or the alloy of which the detachment element (3) is to be manufactured is subjected to a heat treatment.

15. Device according to any one of the claims 1 to 14, **characterized in that** said device is provided in combination with a micro-catheter.

## Revendications

1. Dispositif comprenant un implant endovasculaire (1), qui est prévu pour introduction dans des vaisseaux sanguins ou des cavités corporelles du corps humain ou animal, et un guide (2), l'implant (1) et le guide (2) étant assemblés l'un à l'autre par l'intermédiaire d'un élément détachable (3), lequel est configuré de façon à subir une corrosion électrolytique, de telle sorte que, après introduction de l'implant (1) dans le corps, il en résulte sous l'effet de l'application d'une tension une dissolution au moins partielle de l'élément détachable (3) et un détachement de l'implant (1) à partir du guide (2), **caractérisé en ce que** l'élément détachable (3) est fabriqué en un alliage cobalt-chrome, contenant au moins 20 % en poids de cobalt et 10 à 40 % en poids de chrome.

2. Dispositif selon la revendication 1, **caractérisé en ce que** l'alliage cobalt-chrome contient jusqu'à 70 % en poids de cobalt.

3. Dispositif selon la revendication 2, **caractérisé en ce que** l'alliage cobalt-chrome contient 30 à 60 % en poids de cobalt.

4. Dispositif selon l'une des revendications 1 à 3, **caractérisé en ce que** l'alliage cobalt-chrome contient 15 à 30 % en poids de chrome.

5. Dispositif selon l'une des revendications 1 à 4, **caractérisé en ce que** l'alliage cobalt-chrome contient 4 à 12 % en poids de molybdène.

6. Dispositif selon l'une des revendications 1 à 5, **caractérisé en ce que** l'alliage cobalt-chrome contient 8 à 40 % en poids de nickel.

7. Dispositif selon l'une des revendications 1 à 6, **caractérisé en ce que** l'alliage cobalt-chrome contient 5 à 20 % en poids de tungstène.

8. Dispositif selon l'une des revendications 1 à 7, **caractérisé en ce que** l'alliage cobalt-chrome contient jusqu'à 30 % en poids de manganèse.

9. Dispositif selon l'une des revendications 1 à 8, **caractérisé en ce que** l'alliage cobalt-chrome contient jusqu'à 30 % en poids de fer.

10. Dispositif selon l'une des revendications 1 à 9, **caractérisé en ce que** l'implant (1) est une spirale d'occlusion, un stent, un système extracteur de stent détachable ou un système analogue à un stent pour influer sur la circulation sanguine.

11. Dispositif selon l'une des revendications 1 à 10, **caractérisé en ce que** l'élément détachable (3) est constitué d'un ou plusieurs fils.

12. Dispositif selon l'une des revendications 1 à 11, **caractérisé en ce que** l'élément détachable (3) présente une surface rugueuse.

13. Dispositif selon l'une des revendications 1 à 12, **caractérisé en ce que** l'élément détachable (3) est pré-corrodé.

14. Dispositif selon l'une des revendications 1 à 13, **caractérisé en ce que** l'élément détachable (3) ou l'alliage servant à la fabrication de l'élément détachable (3) est soumis à un traitement thermique.

15. Dispositif selon l'une des revendications 1 à 14, **caractérisé en ce que** le dispositif se présente en combinaison avec un micro-cathéter.
